(19) Europäisches Patentamt
European Patent Office
Office européen des brevets

(11) Numéro de publication: **0 043 598**
**B1**

(12) **FASCICULE DE BREVET EUROPEEN**

(45) Date de publication du fascicule du brevet: **11.07.84**

(51) Int. Cl.[3]: **C 07 D 307/68, C 07 D 307/71**

(21) Numéro de dépôt: **81200543.7**

(22) Date de dépôt: **21.05.81**

(54) **Procédé de transformation de furfural ou de nitro-5 furfural en furonitrile ou en nitro-5 furonitrile.**

(30) Priorité: **09.07.80 FR 8015335**

(43) Date de publication de la demande:
**13.01.82 Bulletin 82/2**

(45) Mention de la délivrance du brevet:
**11.07.84 Bulletin 84/28**

(84) Etats contractants désignés:
**AT BE CH DE GB IT LI LU NL SE**

(56) Documents cités:
**CHEMICAL ABSTRACTS, vol. 84, no. 1, 5. janvier 1976, page 391, ref. 4614k Columbus, Ohio, US J. LIEBSCHER et al.: "Simple synthesis of nitriles from aldehydes"**

**CHEMICAL ABSTRACTS, vol. 80, no. 25, 24 Juin 1974, page 415, ref. 145853s Columbus, Ohio, US**

(73) Titulaire: **AGRIFURANE S.A.**
**Z.I. de Boé Cédex 4412**
**F-47240 Bon Encontre (FR)**

(72) Inventeur: **Gaset, Antoine**
**75 Allée de Brienne**
**F-31000 Toulouse (FR)**
Inventeur: **Gorrichon, Jean-Pierre**
**30 rue des Cèdres**
**F-31400 Toulouse (FR)**
Inventeur: **Audoye, Paul**
**Rue Jean Moulin**
**F-09400 Tarascon-sur-Ariège (FR)**

(74) Mandataire: **Barre, Philippe**
**Cabinet Barre-Gatti-Laforgue 95 rue des Amidonniers**
**F-31069 Toulouse Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 043 598 B1

## Description

L'invention concerne un procédé de transformation de furfural ou de nitro-5 furfural en furonitrile ou en nitro-5 furonitrile.

Le furanne carboxaldéhyde-2, couramment appelé "furfural", possède la formule chimique suivante:

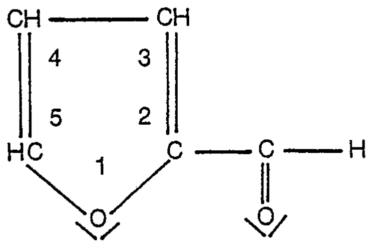

On sait que le furonitrile et son dérivé 5-nitro sont des produits de base utilisés dans de multiples synthèses, notamment dans les industries pharmaceutiques, la chimie des polymères et matières synthétiques et les secteurs agrochimiques. Or, le furfural et son dérivé 5-nitro sont des composés organiques issus des agroressources et qui proviennent souvent de leurs déchets ou sous-produits. On conçoit dans ces conditions l'intérêt économique considérable de la chimie de ces corps et notamment des procédés permettant de transformer ces aldéhydes d'origine végétale qui viennent ainsi se substituer aux dérivés pétrochimiques, pour permettre la synthèse des furonitriles correspondants à partir desquels de nombreuses synthèses organiques peuvent être effectuées.

Parmi les différentes voies de synthèses possibles des furonitriles, il existe un type de procédés faisant intervenir un sel d'hydroxylamine; la présente invention concerne un procédé de ce type.

La plupart des procédés de ce type actuellement mis en oeuvre exige, pour la synthèse du furonitrile ou de ses dérivés, deux étapes, la première consistant à obtenir et à isoler l'aldoxime (notamment par recristallisation), et la seconde réalisant la transformation de l'aldoxime ainsi séparée en furonitrile correspondant, qui peut être effectuée par plusieurs types de traitement où interviennent un ou des réactifs supplémentaires. Ce processus de synthèse est de mise en oeuvre lourde, délicate et relativement onéreuse, en raison de la double étape nécessaire, des opérations d'isolement et de purification de l'aldoxime et de l'addition de réactifs supplémentaires dans la deuxième étape, qui sont généralement non recyclables; en outre, les rendements de chaque étape ne sont pas quantitatifs, ce qui limite notablement le rendement global de la transformation.

De plus, dans ces procédés, le furonitrile formé aux termes des deux étapes sus-évoquées est le plupart du temps présent dans un milieu réactionnel complexe et son extraction de ce milieu en est parfois rendue délicate.

Par ailleurs, on a proposé dans un domaine voisin concernant les aldéhydes aromatiques à 6 atomes diversement substitués (tel que le benzaldéhyde et quelques uns de ses principaux dérivés) un procédé de transformation en une seule étape de l'aldéhyde en nitrile correspondant. On pourra se reporter à cet égard au brevet japonais n° 56450/1972 déposé le 8 juin 1972 et publié sous le n° 14442/1974. Ce type de procédé consiste à faire réagir l'aldéhyde et le sel d'hydroxylamine à une température de 120°C en présence d'un solvant organique spécifique, choisi parmi les corps suivants: acétonitrile, diméthylsulfoxide (D.M.S.O.), diméthylformamide (D.M.F), éthylènecyanohydrine et propionitrile.

Toutefois, ce type de procédés n'est pas transposable en pratique à la transformation du furfural (comme le démontreront les exemples indiqués plus loin) et est donc inapte à permettre à l'homme de l'art de résoudre le problème de la synthèse du furonitrile ou de son dérivé 5-nitro dans des conditions économiques satisfaisantes.

Il convient par ailleurs de remarquer que, même appliqué aux aldéhydes aromatiques à 6 atomes, ce type de procédé présente le grave inconvénient d'exiger des durées de réaction importante (de l'ordre de 3 à 4 heures) de sorte que leur coût est élevé. De plus, ce type de procédé est obligatoirement mis en oeuvre sous atmosphère inerte (en particulier azote) et, pour certains solvants tels que l'acétonitrile et le propionitrile, sous pression.

La présente invention se propose d'indiquer un procédé de transformation qui permettre de transformer en une seule étape le furfural ou son dérivé 5-nitro, en furonitrile ou dérivé dans de remarquables conditions économiques, sur le plan des rendements obtenus et des durées réactionnelles.

Un objectif de l'invention est en particulier de transformer totalement et directement le furfural en furonitrile sans obtenir le corps intermédiaire très stable habituellement obtenu dans la chimie du furfural, à savoir la furfuraloxime.

Un autre objectif est d'indiquer un procédé dans lequel la réaction de synthèse entre le furfural et le sel d'hydroxylamine est quantitative ou sensiblement quantitative, de sorte que ces corps sont consommés intégralement sans résidus, le rendement étant proche de 100%.

Un autre objectif est d'indiquer un procédé ne suscitant aucune dégradation du milieu réactionnel et notamment aucun charbonnage, avec un "solvant réactif" susceptible d'être entièrement recyclé sans perte sensible.

Un autre objectif est de permettre une synthèse du furfural ou de son dérivé 5-nitro dans des laps de temps très courts, inférieurs à 1 heure (et même de l'ordre de quelques minutes en portant la température à des valeurs appropriées).

Un autre objectif est d'indiquer un procédé dont la mise en oeuvre puisse être effectuée à la

presion atmosphérique ambiante.

A cet effect, le procédé de transformation conforme à l'invention du type dans lequel le furfural ou le nitro-5 furfural est mis en présence avec un sel d'hydroxylamine, est caractérisé en ce que la synthèse du furonitrile ou du nitro-5 furonitrile est réalisée en une seule étape en mettant en présence le furfural ou le nitro-5 furfural et le sel d'hydroxylamine dans de la N-méthyl-pyrrolidone (N.M.P.), les corps en présence étant portés à une température comprise entre 120°C et 165°C.

Les expérimentations ont montré de façon inattendue que ce solvant fournissait dans le cas de la transformation du furfural ou de son dérivé 5-nitro, des performances exceptionnelles sur le plan du rendement et de la rapidité de synthèse, et ce, à pression atmosphérique ambiante. De plus, on n'observe aucune dégradation dans le milieu réactionnel, et la N.M.P. utilisée se retrouve intégralement en fin de réaction.

Selon un mode de mise en oeuvre préféré, le sel d'hydroxylamine, qui est avantageusement constitué par du chlorhydrate d'hydroxylamine, et le furfural ou son dérivé 5-nitro sont mis en présence dans la N.M.P., en quantités relatives stoechiométriques.

Dans ces conditions, compte-tenu du caractère quantitatif de la réaction et de la non dégradation du milieu, la phase organique est constituée en fin de réaction par du furonitrile seul (ou dérivé 5-nitro) en solution dans la seule N.M.P. On peut séparer le furonitrile notamment par distillation sous vide partiel et recycler intégralement la N.M.P.

La durée de réaction est fonction inverse de la température et, dans la plage de 120° à 165°C, cette durée est comprise entre 60 mn et 5 mn.

Les exemples 1, 2 décrits ci-après sont destinés à illustrer le procédé de l'invention, cependant que les performances inattendues de ce procédé sont illustrées, par comparaison, par les exemples 3 et 4, qui décrivent des expérimentations menées par les inventeurs sur le furfural avec des solvants du type de ceux préconisés pour les aldéhydes aromatiques à 6 atomes dans le brevet japonais déjà cité.

Ainsi, sera mis en évidence le rôle surprenant que joue la N.M.P. dans la réaction du furfural avec le sel d'hydroxylamine.

### Exemple 1

Synthèse du cyano-2 furanne (furonitrile à partir du furanne carboxaldéhyde-2 (furfural).

Dans un réacteur, on place 0,01 mole de furfural et 0,013 mole de chlorhydrate d'hydroxylamine en solution dans 25 cm3 de N-méthyl pyrrolidone (conditions standards).

Le milieu est alors agité à la pression atmosphérique ambiante:

. soit pendant 10 mn à la température de 160°C,

. soit pendant 30 mn à la température de 140°C,

. soit pendant 60 mn à la température de 120°C.

Ces conditions permettent une transformation totale du furfural en furonitrile (analyse en C.P.V. sur colonne OV—17, et identification en spectrométrie de résonance magnétique nucléaire).

On peut constater en fin de réaction, après extraction classique de la phase organique, une disparition totale du furfural et une absence complète de la furfuraloxime, (qui est le corps stable intermédiaire habituellement obtenu).

Le furonitrile est extrait par distillation sous vide partiel (température d'ébullition: 38°C sous 14 mm/Hg, soit 1841 pascals).

### Exemple 2

Synthèse du nitro-5 furonitrile à partir du nitro-5 furanne carboxaldéhyde-2.

On prépare une solution contenant 0,01 mole de nitro-5 furfural et 0,013 mole de chlorhydrate d'hydroxylamine dans 25 cm3 de N-méthyl pyrrolidone.

On porte à 140°C et on agite pendant 30 mn à la pression atmosphérique ambiante. Les résultats sont comparables sur le plan du rendement à ceux de l'exemple 1.

Le nitro-5 furonitrile est extrait à l'éther et cristallisé (point de fusion: 65°C).

### Exemple Comparatif N° 3

On réitère la synthèse de l'exemple 1 dans des conditions expérimentales analogues, mais en utilisant comme solvant la diméthylformamide (D.M.F.) au lieu de la N.M.P. préconisée par la présente invention.

Les résultats sont résumés dans le tableau ci-après:

| Transformation du furfural en présence de DMF | | | |
|---|---|---|---|
| Température | Durée de réaction | Pourcentage de furonitrile obtenu | Pourcentage de furfuraloxime |
| 120°C | 60 mn | 20% | environ 70% |
| 140°C | 30 mn | 25% | environ 65% |
| 156°C | 10 mn | 25% | environ 55% |

Il est à noter que la température d'ébullition de la D.M.F. est de 156° à pression normale de sorte que la température de 160°C ne peut être atteinte.

On constate en premier lieu un faible pourcentage de furonitrile obtenu quelles que soient les conditions: on obtient surtour la furfuraloxime, produit majoritaire en fin de réaction. De plus, on observe une dégradation du milieu réactionnel qui augmente avec la température; les charbonnages sont importants à 156°C.

Une telle réaction est tout à fait inapte en pratique à permettre une préparation du furonitrile dans des conditions économiques satisfaisantes.

Exemple Comparatif N° 4

On réitère la synthèse de l'exemple 1 en utilisant comme solvant l'acétonitrile à sa température d'ébullition (82°C) à la pression atmosphérique ambiante.

Au bout de 60 minutes, on constate que 90% de furfural n'ont pas été transformés et que les 10% ayant réagi se retrouvent pour la majeure partie sous forme de furfuraloxime. On obtient au bout de ce laps de temps un pourcentage de furonitrile qui n'est pas quantitativement significatif (inférieur à 4%).

Une telle réaction est tout à fait inapte à permettre une préparation industrielle du furonitrile.

**Revendications**

1. Procédé de transformation de furfural ou de nitro-5 furfural en furonitrile ou en nitro-5 furonitrile, du type dans lequel le furfural ou le nitro-5 furfural est mis en présence d'un sel d'hydroxylamine, caractérisé en ce que la synthèse du furonitrile ou du nitro-5 furonitrile est réalisée en une seule étape en mettant en présence le furfural ou le nitro-5 furfural et le sel d'hydroxylamine dans de la N-méthyl pyrrolidone (N.M.P.), les corps en présence étant portés à une température comprise entre 120°C et 165°C.

2. Procédé de transformation selon la revendication 1, caractérisé en ce que le mélange furfural ou nitro-5 furfural, sel d'hydroxylamine et N.M.P. est porté à une température comprise entre 120° et 165°C pendant un laps de temps compris entre 60 mn et 5 mn de durée fonction inverse de la température.

3. Procédé de transformation selon l'une des revendications 1 ou 2, dans lequel le furfural ou le nitro-5 furfural et le sel d'hydroxylamine sont mis en présence dans la N.M.P., en quantités relatives stoechiométriques.

4. Procédé de transformation selon l'une des revendications 1, 2 ou 3, caractérisé en ce que le furonitrile ou nitro-5 furonitrile formé in situ est séparé du solvant par distillation.

5. Procédé de transformation selon l'une des revendications 1, 2, 3 ou 4, dans lequel on utilise comme sel le chlorhydrate

**Claims**

1. Process for the transformation of furfural or 5-nitro-furfural into furonitrile or 5-nitro-furonitrile, in which furfural or 5-nitro-furfural is brought into contact with a hydroxylamine salt, characterised in that the synthesis of furonitrile or 5-nitro-furonitrile is achieved in a single step, by bringing the furfural or 5-nitro-furfural into contact with the hydroxylamine salt in N-methyl pyrrolidone (N.M.P.), the mixed substances being taken to a temperature of between 120°C and 165°C.

2. Transformation process according to Claim 1, characterised in that the mixture of furfural or 5-nitro-furfural, hydroxylamine salt and N.M.P. is taken to a temperature of between 120° and 165°C for a period of time of between 60 minutes and 5 minutes, the duration being inversely proportional to the temperature.

3. Transformation process according to either Claim 1 or 2, in which the furfural or 5-nitro-furfural is brought into contact with the hydroxylamine salt in N.M.P. in stoichiometric relative quantities.

4. Transformation process according to one of the Claims 1, 2 or 3, characterised in that the furonitrile or 5-nitro-furonitrile, formed in situ, is separated from the solvent by distillation.

5. Transformation process according to one of the Claims 1, 2, 3 or 4, in which the salt used is hydroxylamine hydrochloride.

**Patentansprüche**

1. Verfahren zum Umsetzen von Furfural oder 5-Nitro-Furfural in Furonitril oder in 5-Nitro-Furonitril, bei welchem das Furfural oder das 5-Nitro-Furofural mit einem Hydroxylaminsalz zusammengebracht wird, dadurch gekennzeichnet, dass die Synthese des Furonitrils oder des 5-Nitro-Furonitrils in einer einzigen Stufe erreicht wird, wobei das Furfural oder das 5-Nitro-Furfural mit dem Hydroxylaminsalz in N-Methylpyrrolidon (N.M.P.) zusammengebracht wird und wobei die zusammengebrachten Stoffe auf eine zwischen 120°C und 165°C liegende Temperatur gebracht werden.

2. Umsetzungsverfahren nach Anspruch 1, dadurch gekennzeichnet, dass das Gemisch von Furfural oder 5-Nitro-Furfural, Hydroxylaminsalz und N.M.P. auf eine zwischen 120° und 165°C liegende Temperatur während einer zwischen 60 Minuten und 5 Minuten liegenden Zeitspanne gebracht wird, wobei die Dauer im umgekehrten Verhältnis zur Temperatur steht.

3. Umsetzungsverfahren nach einem der Ansprüche 1 oder 2, bei welchem das Furfural oder das 5-Nitro-Furfural mit dem Hydroxylaminsalz in stöchiometrischem Gewichtsverhältnis im N.M.P. zusammengebracht werden.

4. Umsetzungsverfahren nach einem der Ansprüche 1, 2 oder 3, dadurch gekennzeichnet, dass das in situ gebildete Furonitril oder 5-Nitro-Furonitril durch Destillieren vom Lösungsmittel abgetrennt wird.

5. Umsetzungsverfahren nach einem der Ansprüche 1, 2, 3 oder 4, bei welchem als Salz Hydroxylaminchlorhydrat verwendet wird.